# FASCICULE DE BREVET EUROPEEN

(11) **EP 1 785 140 B1**
(45) Date de publication et mention de la délivrance du brevet: **13.01.2010**
(21) Numéro de dépôt: 06291738.0
(22) Date de dépôt: 08.11.2006
(51) Int. Cl.: A61K 31/721, A61K 31/047, A61P 13/00

(54) **Composition et kit pour le traitement des troubles urinaires**
Komposition und Kit für die Behandlung von Harnstörungen
Composition and kit for the treatment of urinary disorders

(30) Priorité: 09.11.2005 FR 0511427
(43) Date de publication de la demande: 16.05.2007
(73) Titulaire: ROQUETTE FRERES, 62136 Lestrem (FR)
(72) Inventeur: Wils, Daniel, 59190 Morbecque (FR); Deremaux Laëtitia, 59800 Lille (FR)
(74) Mandataire: Boulinguiez, Didier

(56) Documents cités:
- EP-A- 1 006 128
- EP-A- 1 369 432
- WO-A-00/64281
- WO-A-92/02149
- WO-A-2005/039518
- WO-A-2006/118370
- WEESE J S ET AL: "Oxalate degradation by intestinal lactic acid bacteria in dogs and cats" VETERINARY MICROBIOLOGY 14 JUL 2004 NETHERLANDS, vol. 101, no. 3, 14 juillet 2004 (2004-07-14), pages 161-166, XP002420699 ISSN: 0378-1135
- THAMILSELVAN S ET AL: "Effect of vitamin E and mannitol on renal calcium oxalate retention in experimental nephrolithiasis." INDIAN JOURNAL OF BIOCHEMISTRY & BIOPHYSICS JUN 1997, vol. 34, no. 3, juin 1997 (1997-06), pages 319-323, XP009079226 ISSN: 0301-1208

## Description

L'invention a pour objet de nouvelles compositions diurétiques et appétentes qui peuvent être utilisées comme médicaments ou comme suppléments alimentaires pour le traitement ou la prévention des troubles urinaires notamment chez les animaux domestiques, et en particulier chez le chat, le chien ou le rat.

Les animaux domestiques et notamment le chat et le chien sont sujets à des troubles urinaires accompagnés ou non de calculs, constitués de phosphates ammoniaco-magnésiens (struvite) ou d'oxalates de calcium.

Le chat est physiologiquement prédisposé à présenter des urines concentrées, en raison de son origine désertique et de son peu d'intérêt pour l'abreuvement.

Chez le chien, la récidive de calculs urinaires s'observe lorsqu'une maladie intercurrente augmente le risque d'infection urinaire, facteur favorisant la formation de calculs de struvite, ou chez le chien qui boit peu.

Les calculs rénaux sont majoritairement constitués de sels de calcium et plus rarement de struvites. Ces dernières peuvent être dissoutes par une alimentation induisant un pH urinaire acide (pH inférieur à 6). La dissolution des calculs d'oxalates de calcium est actuellement impossible et les calculs doivent être extraits de la vessie par voie chirurgicale.

La première recommandation donnée aux sujets souffrant de calculs urinaires est de boire davantage pour diluer les urines. Cette dilution agit à deux niveaux : d'abord en réduisant la concentration de l'urine en électrolytes, ensuite en augmentant la fréquence des mictions et donc en réduisant le temps de séjour de l'urine dans la vessie.

Chez l'animal, le plus difficile est d'augmenter l'abreuvement spontané, en particulier chez le chat qui ne boit en général que 30 millilitres d'eau par kilo de poids corporel.

L'apport d'un aliment humide permet de faire boire un animal peu buveur, mais il n'est pas suffisant, soit parce qu'il ne fait pas ingérer assez d'eau, soit parce qu'il n'augmente pas suffisamment la diurèse.

Les animaux présentant un syndrome urologique sont souvent obèses ou en surpoids, en particulier le chat. L'apport de fibres en quantité importante dans l'aliment est une solution de dilution de la densité énergétique de l'ingéré mais peut augmenter la part d'eau fécale plutôt que la fréquence de miction. Par ailleurs, l'apport d'un aliment de densité énergétique élevée n'est pas souhaitable (risque d'obésité par surconsommation) mais actuellement une quasi-obligation pour ne pas augmenter l'eau fécale.

Ainsi, lorsqu'on souhaite traiter ou prévenir les troubles urinaires, et en particulier les récidives d'urolithiases, l'apport d'un aliment humide est préférable, mais il n'est pas suffisant, peut ne pas être accepté par l'animal, voire induire une pathologie supplémentaire (surpoids, obésité) si la quantité distribuée est mal contrôlée.

Il existe donc aujourd'hui un besoin pour une composition favorisant la quantité d'eau bue, qui favoriserait en même temps l'excrétion urinaire, tout en n'induisant pas de troubles digestifs et en permettant une dilution énergétique de l'ingéré.

Et c'est à l'issue de nombreux travaux de recherche que la Demanderesse a trouvé que ce but pouvait être atteint en préparant une composition diurétique et appétente, comprenant un diurétique osmotique et un agent appétent, particulièrement adaptée au traitement ou à la prévention des troubles urinaires chez l'animal domestique en ce qu'elle augmente considérablement la quantité d'eau bue spontanément par l'animal, tout en favorisant l'excrétion urinaire, et ceci sans déséquilibrer l'apport calorique de l'animal ni entraîner de troubles intestinaux.

La présente invention a pour objet une composition diurétique et appétente, pour l'utilisation dans le traitement ou la prévention des troubles urinaires, comprenant un agent diurétique osmotique et un agent appétent. La présent invention a également pour objet un kit pour l'utilisation dans le traitement ou la prévention des troubles urinaires, comprenant :
- un agent diurétique osmotique ; et
- un agent appétent.
Enfin la présente invention a pour objet l'utilisation d'un agent diurétique osmotique et d'un agent appétent pour la fabrication d'un médicament ou d'un kit destiné au traitement ou à la prévention des troubles urinaires.
Typiquement une composition selon l'invention est faiblement calorique.
Elle peut être par exemple sous la forme d'une boisson, d'un comprimé ou d'une poudre.

On entend par diurétique osmotique une molécule qui agit en augmentant l'osmolarité du filtrat glomérulaire, ce qui provoque un appel d'eau dans le néphron et augmente l'excrétion du sodium et du chlore. On connaît aujourd'hui principalement les polyols comme agents diurétiques osmotiques. Sont particulièrement visés par la présente invention le mannitol, l'érythritol, l'arabitol et l'isosorbide. L'effet diurétique de ces polyols est connu et démontré. Toutefois, ils ne constituent pas à eux seuls une solution au problème soulevé par la Demanderesse, dans le sens où ils ne stimulent pas la soif ou l'abreuvement des animaux.

On entend par agent appétent au sens de la présente invention un agent susceptible d'augmenter la soif ou l'abreuvement spontané de l'animal. Sont concernés par cette définition les dextrines indigestibles et le polydextrose qui induisent, de manière surprenante et inattendue, une envie de boire plus importante de la part de l'animal.

La combinaison de l'agent diurétique et de l'agent appétent selon l'invention, comme il le sera démontré par ailleurs, induit avantageusement une envie de boire et une émission d'urine accrues, tout en préservant l'équilibre énergétique de l'animal, ainsi que son confort intestinal.

L'agent diurétique osmotique est un polyol ou un mélanges de polyols. Le polyol ou mélange de polyols pourra être choisi dans le groupe constitué par le mannitol, l'érythritol, l'arabitol, l'isosorbide ou un mélange de ceux-ci.

L'agent appétent est choisi parmi les dextrines indigestibles, le polydextrose et un mélange de ceux-ci.
Le ratio agent diurétique/agent appétent est de préférence compris entre 1 :50 et 50 :1, de préférence compris entre 1 :20 et 20 :1, plus préférentiellement compris entre 1 :10 et 10 :1, en encore plus préférentiellement compris entre 1 :2 et 1 :3.

La composition ou les différents élements du kit selon l'invention sont destinés à être administrés, de préférence, sous forme de boisson. Selon une autre variante de l'invention, ils peuvent être formulés de manière à pouvoir être incorporés à la ration alimentaire.

La composition ou les différents éléments du kit peuvent se présenter sous différentes formes :
- un sirop à reconstituer dans l'eau ;
- une poudre, ou un comprimés, à diluer dans l'eau ;
- une boisson prête à l'emploi.
Typiquement la poudre poura être enveloppée dans un sachet.

Une boisson diurétique et appétente selon la présente invention comprend un mélange d'au moins un agent diurétique et osmotique et d'au moins un agent appétent, dans un ratio en poids compris entre 1 :50 et 50 :1, de préférence entre 1 :20 et 20 :1, et mieux encore entre 1 :10 et 10 :1.

La concentration de cette boisson sera comprise entre 2 et 750 grammes de matière sèche par litres de boisson, suivant les polyols choisis, leur tolérance, l'animal concerné et son métabolisme, son poids, ainsi qu'en fonction de la tolérance de l'agent appétent.

Selon une variante préférée, ladite boisson comprend de 1 à 50 grammes par litre de polyol diurétique, de préférence de 1 à 40 g/l et encore plus préférentiellement de 2 à 10 g/l de boisson. Certains polyols étant plus ou moins bien tolérés selon les métabolismes et les organismes, les concentrations précitées seront donc ajustées par l'homme du métier de manière à respecter un seuil de tolérance adapté.

Les agents diurétiques osmotiques seront choisis parmi le mannitol, l'érythritol, l'arabitol ou l'isosorbide, seuls ou en mélange entre eux.

En ce qui concerne l'agent appétent, il sera présent dans la boisson de manière à respecter les ratios précités. A titre d'exemple, pour une quantité de 2 grammes par litre de polyol, la boisson pourra comprendre de 0,2 à 20 grammes d'agent appétent par litre de boisson en fonction de l'effet recherché et de l'animal concerné.

L'agent appétent sera choisi dans le groupe constitué par les dextrines indigestibles et le polydextrose, seuls ou en mélange entre eux. Les dextrines indigestibles selon la présente invention désignent notamment les dextrines de blé, de maïs, de pois ou de pomme de terre, obtenues par grillage d'amidon à sec et en milieu acide de manière à obtenir des dextrines hautement branchées telles que notamment celles commercialisées sous l'appellation FIBERSOL® par la société MATSUTANI, ou encore les maltodextrines branchées commercialisées par la Demanderesse et décrites dans le brevet EP 1.006.128. L'on utilisera de préférence des maltodextrines branchées présentant un poids moléculaire en nombre compris entre 2000 et 3000 grammes par mole et un taux de liaisons glucosidiques 1->6 compris entre 15 et 35%. Les variantes hydrogénées desdites dextrines indigestibles, ainsi que les sirops de polyols en contenant, comme notamment les sirops de maltitol sont également concernées par la présente invention.

Le polydextrose est un polymère de glucose faiblement calorique, connu de l'homme du métier, et obtenu par réaction de polycondensation de glucose, maltose, oligomères de glucose, ou hydrolysats d'amidon, en présence d'acide. Le terme « polydextrose » selon la présente invention comprend également les variantes purifiées et/ou hydrogénées du polydextrose.

La Demanderesse a mis en évidence au cours d'études chez le rat notamment, que de manière surprenante et inattendue, le polydextrose et les dextrines indigestibles augmentaient de façon très significative les quantités d'eau bue par les animaux. Par contre, la surconsommation d'eau générée par ces agents appétents n'a pas montré de conséquences sur la diurèse, mais sur la matière sèche des fécès. L'excès d'eau consommée est donc excrété par voie fécale.

Par contre, si l'on associe un agent diurétique et un agent appétent susceptible d'augmenter l'abreuvement, l'excrétion urinaire est alors favorisée et le problème technique à la base de la présente invention est résolu, d'autant plus avantageusement que ladite association n'induit pas de troubles digestifs et permet une dilution énergétique de l'ingéré, ce qui constitue une solution idéale.

Lorsque l'on choisit de préparer une boisson, on mettra en oeuvre de préférence une concentration en agent diurétique supérieure à 1 g/1. En deçà de 1 g/l de polyol diurétique dans la composition, l'effet diurétique faible. Au-delà de 50 g/l de polyol diurétique, les risques de troubles intestinaux deviennent non négligeables, cette limite dépendant toutefois du polyol concerné. Cette concentration sera bien entendu déterminée également en fonction de la tolérance aux polyols de l'animal concerné, celle-ci étant variable d'une espèce à l'autre. Selon un mode de réalisation préféré, on choisira des concentrations comprises entre 2 et 10 grammes par litre de polyol diurétique.

En ce qui concerne l'agent appétent, celui-ci est plus efficace en tant que tel à partir de 2 grammes par litres ; au-delà de 20 grammes par litres, il risque d'entraîner des troubles intestinaux (diarrhées, ballonnements). De préférence, on choisira des concentrations comprises entre 3 et 15 grammes par litre, ou mieux encore comprises entre 5 et 10 grammes par litre.

Chez le chat et le chien, des résultats très significatifs ont été obtenus avec une boisson comprenant 2 grammes par litres de polyol diurétique, et 5 grammes par litres d'agent appétent (soit un ratio en poids de 1 :2,5). De très bons résultats sont généralement obtenus avec des ratios en poids compris entre 1 :2 et 1 :3.

Lorsque la composition selon l'invention consiste en un sirop concentré à reconstituer dans l'eau, on fera comporter au sirop les quantités nécessaires d'agent diurétique et d'agent appétent de manière à ce que la boisson finale comprenne des proportions efficaces du mélange. Ce sirop pourra comprendre de 2 à 750 grammes par litre, de préférence de 2 à 500 g/litre de matière sèche dudit mélange agent diurétique/agent appétent, l'homme du métier choisira aisément en fonction de son utilisation les proportions qui conviennent, en tenant compte des seuils de tolérance de chaque composé du mélange.

A titre d'exemple, un sirop concentré selon l'invention, que l'on reconstituera à raison d'un volume pour 70 volumes d'eau, comprendra avantageusement 140 g/litre de polyol diurétique et 350 g/l d'agent appétent. On pourra par exemple préparer 350 ml de boisson à partir de 5 ml de sirop selon l'invention, ce qui peut correspondre avantageusement à un bouchon de sirop à reconstituer dans un flacon de 350 ml de contenance finale.

Le sirop concentré selon l'invention sera suffisamment concentré pour éviter la croissance de microorganismes lors de son stockage. Il faudra à titre d'exemple se placer à des valeurs d'eau libre aw inférieures à 0,85 ou ajouter un conservateur. En tout état de cause, il conviendra de respecter le ratio en poids polyol/agent appétent compris entre 1 :50 à 50 :1, et mieux entre 1 :20 et 20 :1.

Selon une autre variante de la présente invention, la composition ou les différents éléments du kit se présentent sous forme de poudre ou de comprimé à reconstituer dans l'eau ou à mélanger à la ration alimentaire.

Une telle poudre comprendra le ratio précité de polyol et d'agent appétent Par exemple, pour un sachet de poudre à reconstituer dans 1 litre d'eau, le sachet-dose comprendra de 1 à 4 grammes de polyol diurétique, et de 2 à 20 grammes d'agent appétent. Selon une variante préférée, il comprendra 2 grammes de polyol et 5 grammes d'agent appétent.

De même, pour la formulation de comprimés, on pourra préparer des comprimés comprenant un ratio de 1 :50 à 50 :1 de polyols diurétiques et d'agent appétent. A titre d'exemple, un comprimé de 1 gramme pourra comprendre 350 mg de polyol et 715 mg d'agent appétent.

De très bons résultats ont été obtenus avec les compositions suivantes :
- 2 g/litres de mannitol et 5 g/litres de dextrines indigestibles ;
- 2 g/litres d'érythritol et 5 g/litres de polydextrose ;
- 2 g/litres d'isosorbide et 5 g/litres de dextrines indigestibles ou de polydextrose ;
- 2,5 g/litres d'arabitol et 5 g/litres de dextrines indigestibles ou de polydextrose ;
- Boisson comprenant un mélange de mannitol et d'érythritol, ainsi que des dextrines indigestibles.

Bien entendu, la composition ou les différents éléments du kit selon l'invention pourront comprendre tout additif approprié notamment à la préparation de boissons, comme des arômes, conservateurs, vitamines, minéraux, principes actifs, dès lors qu'ils ne dénaturent pas les effets de ladite composition ou dudit kit.

La présente invention vise également l'utilisation d'un agent diurétique osmotique et d'un agent appétent pour la fabrication d'un médicament ou d'un kit destiné au traitement ou à la prévention des troubles urinaires, notamment chez les animaux domestiques, et en particulier pour le traitement des urolithiases. Le traitement consiste en l'administration à un animal domestique d'une quantité efficace de composition diurétique et appétente, sous forme de boisson ou ajoutée à la ration alimentaire, de manière à stimuler l'abreuvement et favoriser la diurèse.

La présente invention embrasse également la variante selon laquelle l'agent diurétique peut être administré simultanément, consécutivement ou préalablement à l'agent appétent pour traiter ou prévenir les troubles urinaires.

Il va sans dire que la composition ou le kit selon l'invention peuvent également trouver des applications chez l'homme, dans le sens où la combinaison d'un agent diurétique et d'un agent augmentant la soif peut être mise à profit, outre pour le traitement ou la prévention des troubles urinaires, dans la formulation de boissons diététiques à visées notamment drainantes, dépuratives et/ou amincissantes.

L'invention sera mieux comprise à la lecture des exemples qui suivent, qui se veulent illustratifs et non limitatifs.

### Exemple 1 : influence de l'administration par voie orale d'érythritol, de mannitol ou d'isosorbide chez le rat Sprague-Dawley.

On administre à un lot de rats par voie orale des doses de polyols diurétiques de manière à mesurer l'effet diurétique desdits polyols et leur influence éventuelle sur la quantité d'eau bue. L'étude est menée sur un lot de trente rats, pendant 4 jours, et les doses d'essai sont administrées à raison de 2ml par animal et par jour de solutions à 250 g/litres de polyols. De l'eau est distribuée ad libitum aux animaux. Les quantités d'urine émise et de boisson consommée sont mesurées par pesée.

Les quantités de boisson sont exprimées en grammes par animal et par jour. La diurèse est exprimée en millilitres en fonction du temps.
Lot A : lot témoin
Lot B : gavage érythritol
Lot C : gavage mannitol
Lot D : gavage isosorbide
Lot E : mélange isosorbide-mannitol 1/1

### CONSOMMATION DE BOISSON (moyennes sur dix rats)

Lot A : la consommation d'eau est de 24,8 g grammes par animal et par jour (moyenne de dix essais)

Lot B : la consommation d'eau est de 17,9 g/animal/jour

Après 3 jours, la consommation d'eau après gavage à l'érythritol n'est pas supérieure au témoin.
Lot A : sur quatre jours, elle est de 11,2 g/animal/jour
Lot C : 13,9
Lot D : 13,1
Lot E : 13,3

La consommation d'eau après gavage au mannitol ou à l'isosorbide est très légèrement supérieure au témoin.

DIURESE (moyennes sur dix rats, en millilitres)

| lot | Avant gavage | 45 min | 1h35 | 3h | 5h | 7h | Diurèse cumulée sur 24h |
|---|---|---|---|---|---|---|---|
| A | 3.2 | 0.9 | 0.6 | 0.4 | 0.7 | 0.6 | 11.7 |
| B (érythritol) | 1.02 | 0.21 | 0.99 | 1.38 | 1.21 | 0.83 | 18.43 |
| C (mannitol) | 3.0 | 0.3 | 0.3 | 0.7 | 0.5 | 1.3 | 16.5 |
| D (isosorbide) | 2.4 | 1.5 | 1.1 | 1.2 | 1.1 | 0.6 | 13.0 |
| E (mélange mannitol/isosorbide) | 1.85 | 0.5 | 0.46 | 1.08 | 1.35 | 1.63 | 16.16 |

Conclusion : on observe une augmentation significative de la diurèse par rapport au témoin pour les essais de gavage aux polyols diurétiques. Dans le cas de l'érythritol, on observe une augmentation de la diurèse dès 3 heures, alors qu'elle est plus tardive pour les autres polyols (entre 3 et 5 heures).

Le gavage des rats au moyen de ces polyols ne permet toutefois pas d'augmenter de façon significative la quantité d'eau bue pendant les essais. Il serait donc nécessaire de compléter le traitement des rats au moyen d'un agent appétent susceptible d'augmenter l'abreuvement spontané des rats, de manière à compenser les pertes hydriques induites par les polyols diurétiques.

### Exemple 2 : étude des effets de l'administration de dextrine indigestible chez le rat.

On incorpore à l'alimentation de rats des doses croissantes de NUTRIOSE®FB (commercialisée par la Demanderesse) de 1.25 à 5% en poids. L'étude est réalisée sur 50 mâles obèses et sur 50 mâles témoins.

Les animaux sont suivis pendant 46 semaines, pendant lesquelles les consommations de boisson et d'aliment sont mesurées.

Rats témoins : consommation totale moyenne de boisson en grammes par animal à l'issue de l'étude :
- Témoin : 5360,3
- 1,25% NUTRIOSE®FB . 5251,0
- 2,5% NUTRIOSE®FB : 5637,2
- 5% NUTRIOSE®FB : 5776,3

Rats obèses :
- témoin : 6206,3
- 1,25% NUTRIOSE®FB : 6885,6
- 2,5% NUTRIOSE®FB : 6283,8
- 5% NUTRIOSE®FB : 6851,0

Conclusion : les rats traitées à 5% de NUTRIOSE®FB consomment plus d'eau que les rats témoins (7% de plus). Ceux traités à 2,5% de NUTRIOSE®FB en consomment 5% de plus.

Chez le rat obèse, cette différence est encore plus marquée (augmentation de 10 à 12%).

Il apparaît clairement que le traitement des rats au NUTRIOSE®FB induit une plus forte consommation d'eau par rapport au témoin. Les rats obèses consomment davantage d'eau que les rats témoins, ce qui s'explique en partie par la consommation d'aliment plus importante de ces derniers.

### Exemple 3 : étude des effets de l'administration de polydextrose chez le rat.

Comme pour l'essai précédent, on incorpore du polydextrose LITESSE® (PFIZER), du NUTRIOSE®FB06 (dextrine de blé) et du NUTRIOSE®FM06 (dextrine de maïs) à l'alimentation de rats Sprague-Dawley durant 21 jours. Les doses d'incorporation de chaque composé sont de 1,25, 2,5 et 5%.

De la même manière, la consommation de boisson totale durant l'étude est mesurée et exprimée en grammes par animal.

Résultats :
- témoin : 484,0
- 1,25% NUTRIOSE®FB06 : 514,9
- 2,5% NUTRIOSE®FB06 : 494,6
- 5% NUTRIOSE®FB06 : 503,8
- 1,25% NUTRIOSE®FM06 : 515,6
- 2,5% NUTRIOSE®FM06 . 520,7
- 5% NUTRIOSE®FM06 : 529,0
- 1,25% LITESSE® : 576,8
- 2,5% LITESSE® : 542,4
- 5% LITESSE® : 582,7

L'introduction de polydextrose dans l'alimentation des rats entraîne une augmentation significative de la consommation d'eau au cours du temps et sur la totalité de l'étude.

Les rats traités au NUTRIOSE® voient également leur consommation d'eau augmenter par rapport au témoin mais de façon moins marquée. Toutefois, les consommations d'eau supérieures n'induisent pas d'augmentation de la diurèse. Par contre, l'étude a mis en évidence que la matière sèche des fécès diminuait au cours de l'étude, ce qui démontre que la surconsommation d'eau est compensée par une élimination fécale et non urinaire de celle-ci.

Les dextrines indigestibles et le polydextrose induisent manifestement et de manière surprenante une augmentation de l'abreuvement des rats, mais n'ont pas d'effet sur la diurèse.

### Exemple 4 : étude des effets de l'administration de mélanges d'agent appétent et d'agent diurétique.

On administre dans la boisson des mélanges de polyols diurétiques et d'agents appétents à un lot de rats. Cette étude a pour but de mesurer l'effet diurétique de ces polyols et l'influence de l'agent appétent sur la quantité de boisson consommée et sur le volume d'urine excrétée. L'étude est menée sur des lots de 10 rats chacun pendant 3 jours. Les produits testés sont administrés à raison de 50g/L pour l'agent appétent et 25g/L pour les polyols. Cette boisson est laissée ad-libitum aux animaux. La boisson consommée est mesurée par pesée chaque jour de l'étude et est exprimée en grammes en fonction du temps. Les diurèses sont exprimées en millilitres.

Organisation des lots :
- Lot 1 : Témoin
- Lot 2 : Polydextrose/Erythritol
- Lot 3 : NUTRIOSE®FB17/Erythritol
- Lot 4 : NUTRIOSE®FB17/Mannitol

Consommation de boisson (moyennes sur dix rats) :

| | J0 à J1 | J1 à J2 | J2 à J3 | Total | % d'évolution par rapport au témoin |
|---|---|---|---|---|---|
| Lot 1 | 27,6 | 30,2 | 30,1 | 87,9 | - |
| Lot 2 | 39,9 | 34,1 | 38,5 | 112,4 | +27% |
| Lot 3 | 39,8 | 37,3 | 40,2 | 117,4 | +33% |
| Lot 4 | 35,4 | 30,7 | 31,4 | 97,5 | +10% |

Diurèses (moyennes sur dix rats) :

| | J0 à J1 | J1 à J2 | J2 à J3 | Total | % d'évolution par rapport au témoin |
|---|---|---|---|---|---|
| Lot 1 | 6,7 | 8,0 | 7,6 | 22,4 | - |
| Lot 2 | 10,4 | 9,6 | 5,6 | 25,6 | +14% |
| Lot 3 | 12,5 | 12,4 | 9,8 | 34,7 | +54% |
| Lot 4 | 10,0 | 9,5 | 6,9 | 26,4 | +17% |

### Conclusion :

On observe une augmentation significative de la quantité de boisson consommée au cours de l'étude pour tous les lots, à savoir les lots : polydextrose/érythritol, NUTRIOSE®FB17/érythritol, NUTRIOSE®FB17/mannitol. Cette augmentation est très importante dès le démarrage de l'étude et se poursuit jusqu'à la fin de l'étude. On observe des augmentations de consommation allant de +10% à +33% pour ces lots sur la totalité de l'étude et en comparaison au lot témoin.

La mesure de la diurèse montre que ces mêmes mélanges entraînent une très importante augmentation du volume d'urines excrétées dès le démarrage de l'étude et ce tout au long de l'étude. Par rapport au lot témoin, ces augmentations sont de l'ordre de +14% à +54% sur le volume total en fin d'étude, par rapport au lot témoin.

Les résultats de consommation de boisson et de diurèse démontrent un lien très étroit entre ces deux paramètres. Les agents appétents de l'étude entraînent très certainement une augmentation de la consommation hydrique et donc du volume d'urine excrétée ; ce second phénomène est accentué par l'effet des agents diurétiques testés.

Il est entendu que les doses testées chez le rat peuvent être supérieures aux doses pouvant être administrées à des chats ou des chiens en fonction de leur tolérance digestive, elles se veulent ici uniquement illustratives.

## Revendications

1. Composition diurétique et appétente, pour l'utilisation dans le traitement ou la prévention des troubles urinaires, comprenant un agent diurétique osmotique choisi parmi les polyols et les mélanges de ceux-ci, et un agent appétent choisi parmi les dextrines indigestibles, le polydextrose et un mélange de ceux-ci.

2. Composition selon la revendication 1, **caractérisée en ce que** elle est faiblement calorique.

3. Composition selon la revendication 1 ou 2, **caractérisée en ce que** la composition est une boisson, un comprimé ou une poudre.

4. Kit pour l'utilisation dans le traitement ou la prévention des troubles urinaires, comprenant :
- un agent diurétique osmotique choisi parmi les polyols et les mélanges de ceux-ci; et
- un agent appétent choisi parmi les dextrines indigestibles, le polydextrose et un mélange de ceux-ci.

5. Utilisation d'un agent diurétique osmotique choisi parmi les polyols et les mélanges de ceux-ci et d'un agent appétent choisi parmi les dextrines indigestibles, le polydextrose et un mélange de ceux-ci pour la fabrication d'un médicament ou d'un kit destiné au traitement ou à la prévention des troubles urinaires.

6. Composition selon l'une quelconque des revendications 1 à 3, ou kit selon la revendication 4 ou utilisation selon la revendication 5, **caractérisés en ce que** le ratio en poids agent diurétique/agent appétent est compris entre 1 :50 et 50 :1.

7. Composition selon l'une quelconque des revendications 1 à 3, kit selon la revendication 4 ou utilisation selon la revendication 6, **caractérisés en ce que** le ratio en poids agent diurétique/agent appétent est compris entre 1 :20 et 20 :1.

8. Composition selon l'une quelconque des revendications 1 à 3, 6, 7, ou kit selon l'une quelconque des revendications 4, 6, 7, ou utilisation selon l'une quelconque des revendications 5 à 7, **caractérisés en ce que** le ratio en poids agent diurétique/agent appétent est compris entre 1 :2 et 1 :3.

9. Composition selon l'une quelconque des revendications 1 à 3, 6 à 8, ou kit selon l'une quelconque des revendications 4, 6 à 8, ou utilisation selon l'une quelconque des revendications 5 à 8, **caractérisés en ce que** ledit polyol ou mélange de polyols est choisi dans le groupe constitué par le mannitol, l'érythritol, l'arabitol, l'isosorbide ou un mélange de ceux-ci.

## Claims

1. Diuretic and appetence-inducing composition for use in the treatment or prevention of urinary disorders comprising an osmotic diuretic agent selected from polyols and mixtures thereof, and an appetence-inducing agent selected from indigestible dextrins, polydextrose and a mixture thereof.

2. Composition according to claim 1, wherein said composition is a low-calorie composition.

3. Composition according to claim 1 or 2, wherein said composition is a drink, a tablet or a powder.

4. Kit for use in the treatment or prevention of urinary disorders comprising
- an osmotic diuretic agent selected from polyols and mixtures thereof, and
- an appetence-inducing agent selected from indigestible dextrins, polydextrose and a mixture thereof.

5. Use of an osmotic diuretic agent selected from polyols and mixtures thereof and of an appetence-inducing agent selected from indigestible dextrins, polydextrose and a mixture thereof, for manufacturing a drug or a kit for the treatment or prevention of urinary disorders.

6. Composition according to any of claims 1 to 3, or kit according to claim 4, or use according to claim 5, wherein the diuretic agent/appetence-inducing agent weight ratio is between 1:50 and 50:1.

7. Composition according to any of claims 1 to 3, or kit according to claim 4, or use according to claim 6, wherein the diuretic agent/appetence-inducing agent weight ratio is between 1:20 and 20:1.

8. Composition according to any of claims 1 to 3, 6, 7, or kit according to any of claims 4, 6, 7, or use according to any of claims 5 to 7, wherein the diuretic agent/appetence-inducing agent weight ratio is between 1:2 and 1:3.

9. Composition according to any of claims 1 to 3, 6 to 8, or kit according to any of claims 4, 6 to 8, or use according to any of claims 5 to 8, wherein said polyol or mixture of polyols is selected from the group consisting of mannitol, erythritol, arabitol, isosorbide, and any mixtures thereof.

## Patentansprüche

1. Diuretische und appetitanregende Zusammensetzung zur Verwendung bei der Behandlung oder der Prävention von Harnstörungen, die ein osmotisches Diuretikum, ausgewählt aus Polyolen und deren Mischungen, und ein appetitanregendes Mittel, ausgewählt aus unverdaulichen Dextrinen, Polydextrose und einer Mischung aus diesen, umfasst.

2. Zusammensetzung nach Anspruch 1, **dadurch gekennzeichnet, dass** sie kalorienarm ist.

3. Zusammensetzung nach Anspruch 1 oder 2, **dadurch gekennzeichnet, dass** die Zusammensetzung ein Getränk, eine Tablette oder ein Pulver ist.

4. Set zur Verwendung bei der Behandlung oder der Prävention von Harnstörungen, das umfasst:
- ein osmotisches Diuretikum, ausgewählt aus Polyolen und deren Mischungen; und
- ein appetitanregendes Mittel, ausgewählt aus unverdaulichen Dextrinen, Polydextrose und einer Mischung aus diesen.

5. Verwendung eines osmotisches Diuretikums, ausgewählt aus Polyolen und deren Mischungen, und eines appetitanregenden Mittels, ausgewählt aus unverdaulichen Dextrinen, Polydextrose und einer Mischung aus diesen, zur Herstellung eines Medikaments oder eines Sets, das zur Behandlung oder zur Prävention von Harnstörungen bestimmt ist.

6. Zusammensetzung nach einem der Ansprüche 1 bis 3, oder Set nach Anspruch 4 oder Verwendung nach Anspruch 5, **dadurch gekennzeichnet, dass** das Gewichtsverhältnis Diuretikum/appetitanregendes Mittel von 1 :50 bis 50 :1 beträgt.

7. Zusammensetzung nach einem der Ansprüche 1 bis 3, Set nach Anspruch 4 oder Verwendung nach Anspruch 6, **dadurch gekennzeichnet, dass** das Gewichtsverhältnis Diuretikum/appetitanregendes Mittel von 1 :20 bis 20 :1 beträgt.

8. Zusammensetzung nach einem der Ansprüche 1 bis 3, 6, 7, oder Set nach einem der Ansprüche 4, 6, 7, oder Verwendung nach einem der Ansprüche 5 bis 7, **dadurch gekennzeichnet, dass** das Gewichtsverhältnis Diuretikum/appetitanregendes Mittel von 1 :2 bis 1 :3 beträgt.

9. Zusammensetzung nach einem der Ansprüche 1 bis 3, 6 bis 8, oder Set nach einem der Ansprüche 4, 6 bis 8, oder Verwendung nach einem der Ansprüche 5 bis 8, **dadurch gekennzeichnet, dass** das Polyol oder die Mischung aus Polyolen ausgewählt ist aus der Gruppe bestehend aus Mannitol, Erythrit, Arabit, Isosorbid und einer Mischung aus diesen.
